# EUROPEAN PATENT APPLICATION

(11) **EP 4 796 037 A1**
(43) Date of publication of application: **26.08.2026**
(21) Application number: 25305238.5
(22) Date of filing: 21.02.2025
(51) Int. Cl.: A61B 5/339, A61B 5/349, A61B 5/00

(54) **METHOD AND SYSTEM FOR FACILITATING THE DIAGNOSTIC OF CARDIAC ABNORMALITIES**

(71) Applicant: Koninklijke Philips N.V., 5656 AE Eindhoven (NL)
(72) Inventor: CHOFFIN, Benoît, 5656 Eindhoven (NL); KHELDOUNI, Amine, 5656 Eindhoven (NL); CURTIT, Sarah, 5656 Eindhoven (NL); SHRESHTHA, Kumar, 5656 Eindhoven (NL); DUPONT, Rémi, 5656 Eindhoven (NL); PEZZOTTI, Nicola, 5656 Eindhoven (NL)
(74) Representative: Philips Intellectual Property & Standards

(57) **Abstract**

The invention relates to a system for facilitating the diagnostic of cardiac abnormalities based on ECG measurements. The system comprises an input interface configured to receive an electrocardiogram ECG signal; analyze the ECG signal using a delineation algorithm so as to segment the ECG signal into multiple beats; determine for each beat a set of measurements; group beats having similar measurements per cluster, each cluster representing a different sub-rhythm; and aggregate measurements within each cluster before being displayed to an user.

## Description

### FIELD OF THE INVENTION

This invention relates to the field of electrocardiogram ECG measurements and more specifically to a method and system for facilitating the diagnostic of cardiac abnormalities based on ECG measurements.

### BACKGROUND OF THE INVENTION

ECGs are a diagnostic modality that is quite compelling as it is relatively easy to acquire signal without expensive devices or lengthy acquisition procedures. The resulting signals can tell a lot about the cardiac status of the patient, making it the ideal entry point of all cardiovascular disease (CVD) care pathways. However, interpreting an ECG is far from being trivial. It requires a deep understanding not only of the electro-physiology of the heart, but also how cardiac abnormalities result in a change in the electrical signal which is recorded.

ECG machines and algorithms typically display ECG measurements based on the dominant beat or rhythm, or an aggregation of the individual measurements of the beats, or an averaged "abstract" representative beat construction. This gives only a partial understanding of the ECG features to the user.

Therefore, this type of aggregation tends to be high-level and blends beats and sub-rhythms potentially arising from different origins in the heart.

### SUMMARY OF THE INVENTION

It is an aim of the invention to provide a method and system, which gives the user a more complete understanding of the ECG.

The invention is defined by the appended claims.

According to examples in accordance with an aspect of the invention, there is provided a method for facilitating the diagnostic of cardiac abnormalities based on ECG measurements.

The method comprises the steps of:
- receiving an ECG signal;
- analyzing the ECG signal using a delineation algorithm so as to segment the ECG signals into multiple beats;
- determining for each beat a set of measurements;
- grouping beats having similar measurements per cluster, each cluster representing a different sub-rhythm;
- aggregating measurements within each cluster before being displayed to a user.

In an embodiment, the method comprises the step of applying an embedding algorithm to generate embedding data representative of the plurality of beats, and where the grouping step is based on beats having a similar morphology.

In another embodiment, the measurements include one or more of QRS duration, QRS axis, QT duration, corrected QT duration, PR duration, ST elevation, RR with preceding beats, P wave axis, and P wave duration.

According to another aspect of the invention, there is provided a computer program comprising code means for implementing the method when said program is run on a processing system.

According to another aspect of the invention, there is provided a system of facilitating the diagnostic of cardiac abnormalities based on ECG measurements.

The system comprises an input interface configured to:
- receiving an electrocardiogram ECG signal;
- analyzing the ECG signal using a delineation algorithm so as to segment the ECG signal into multiple beats;
- determining for each beat a set of measurements;
- grouping beats having similar measurements per cluster, each cluster representing a different sub-rhythm;
- aggregating measurements within each cluster before being displayed to an user.

The system is advantageously included in a monitoring system comprising a cardiovascular monitor.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematical overview of the proposed system in accordance with an embodiment of the invention;
Fig. 2 is a schematical overview of the proposed system in accordance with another embodiment of the invention;
Fig. 3 is an ECG display showing a 10 second 12-lead resting ECG;
Fig. 4 shows a user interface in accordance with invention showing the decomposition of an EGC signal into sub-rhythms; and
Figs 5, 6 and 7 show the results of the method in accordance with the invention with the beats of each of the sub-rhythms plotted along with the measurements averaged over these beats.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

In this invention, there is provided a method for decomposing an ECG into one or multiple sub-rhythms and report ECG measurements in a granular manner, for each of the identified sub-rhythms/beats.

Such a method makes it possible to increase the speed of analysis of an ECG for the operator and give them a more thorough and precise understanding of the ECG.

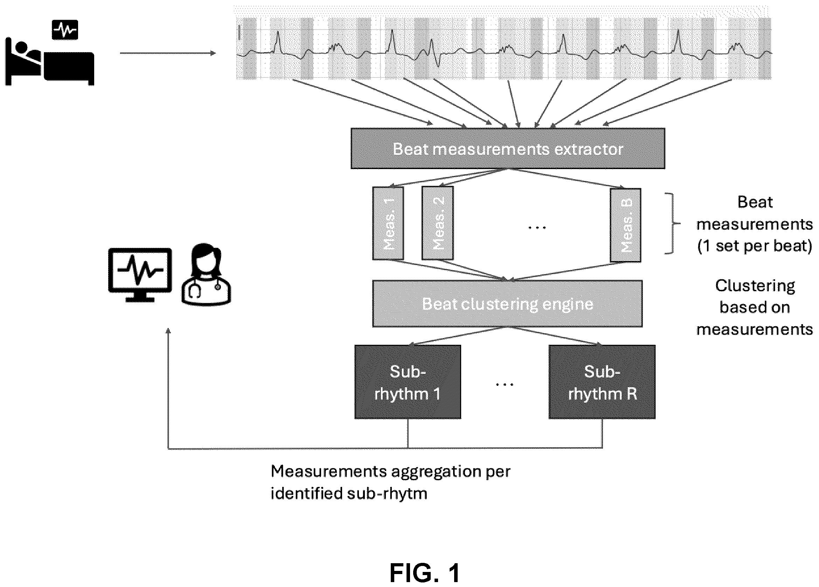

Fig. 1 gives a schematical overview of the proposed method and system in accordance with an embodiment of the invention.

The method takes as input an ECG with delineated beats, i.e. the ECG signal is segmented into multiple beats, and each beat into multiple waves, which are typically P wave, QRS complex and T wave. Then, it computes a set of relevant measurements for each beat.

Once all beats and corresponding measurements are extracted, a clustering algorithm is applied to the measurements and clusters of beats are computed based on these measurements. The clustering algorithm is based on K-means, Hierarchical clustering or the like.

Each cluster corresponds to beats having similar measurements, which serves here as a proxy for concluding that these beats have a similar origin and conduction pathway

Measurements are then aggregated within each cluster, before being displayed to the operator.

In an embodiment of the invention, measurements include one or more of:
- QRS duration;
- QRS axis;
- QT duration ;
- corrected QT (QTc) duration;
- PR duration ;
- ST elevation;
- RR with preceding beats ;
- P wave axis ;
- P wave duration.

If the algorithm detects only one single sub-rhythm, it will only display the global aggregated measurements over all beats of the recording, just as what is implemented and displayed on modern ECG machines.

If the user disagrees with the performed grouping, he can manually change beats from one cluster to another. The measurements are then automatically recomputed and displayed.

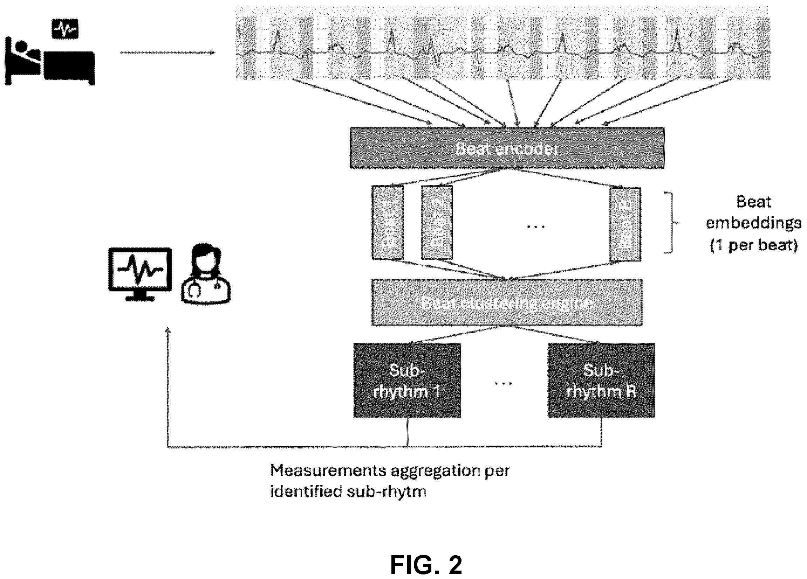

Fig. 2 gives a schematical overview of an improvement of the previous method and system in accordance with another embodiment of the invention.

In this embodiment, a dense embedding is extracted for each of the beats of the recording. The embedding can potentially capture different types of information regarding each beat: for instance, location in the ECG, relationship to other beats, presence/absence of P waves, etc. Here, the embedding captures morphological information about the beat, which serves as a proxy of the origin and the conduction pathway of the beat. A distance metric allows then to compare 2 beat embeddings to determine if the 2 beats are of similar nature or not.

Once all beat embeddings are extracted, a clustering algorithm is applied on these embeddings and clusters of beats are computed.

Each cluster reflects a distinct and coherent beat morphology, reflecting a distinct beat origin and conduction pathway in the heart.

Measurements are then computed and aggregated within each cluster, before being displayed to the operator.

Such a method makes it possible to extract not only the beat measurements but also the beat morphology. This increases the method accuracy since two beats having similar measurements may have different origins.

However, the method disclosed in the embodiment of Fig. 1 has the advantage of a lower complexity.

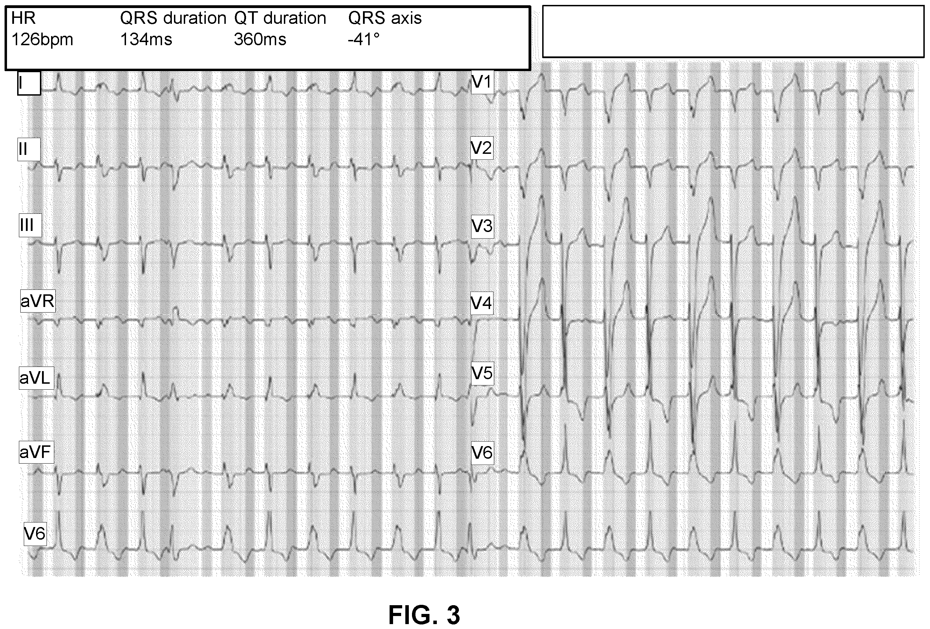

Fig. 3 discloses an ECG display showing a 10 second 12-lead resting ECG.

As it can be seen on the top of Fig. 3, the ECG display system provides the user with a set of measurements, which are the aggregated measurements explained in the background section. These measurements are automatically computed based on the segmentation of the ECG into multiple beats and waves by the ECG analysis system. These measurements are averaged over the whole ECG and do not provide a completely faithful representation of the ECG measurements to the user. For instance, the QRS duration displayed is the mean over all beats and measures 134ms.

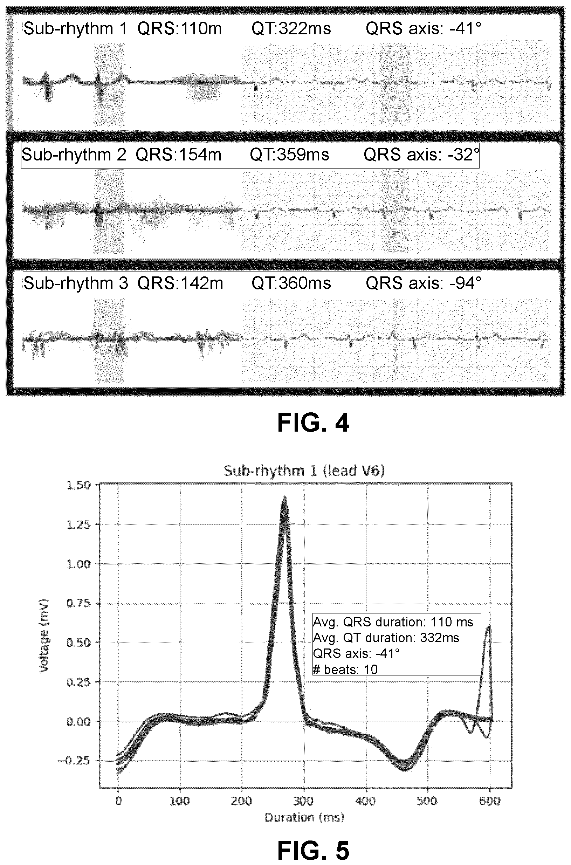

Fig. 4 discloses an example of a user interface of the method in accordance with invention showing the decomposition of the ECG signal into sub-rhythm measurements. This image can be displayed alone or in association with its corresponding ECG signal.

The displayed results are based on the method in accordance with the invention which has been run on the 10 second 12-lead resting ECG signal of Fig. 3. Three sub-rhythms have been identified, and a set of measurements has been associated with each sub-rhythm.

Figs 5, 6 and 7 display the results of the method in accordance with the invention with the beats of each of the sub-rhythms plotted along with the measurements averaged over these beats.

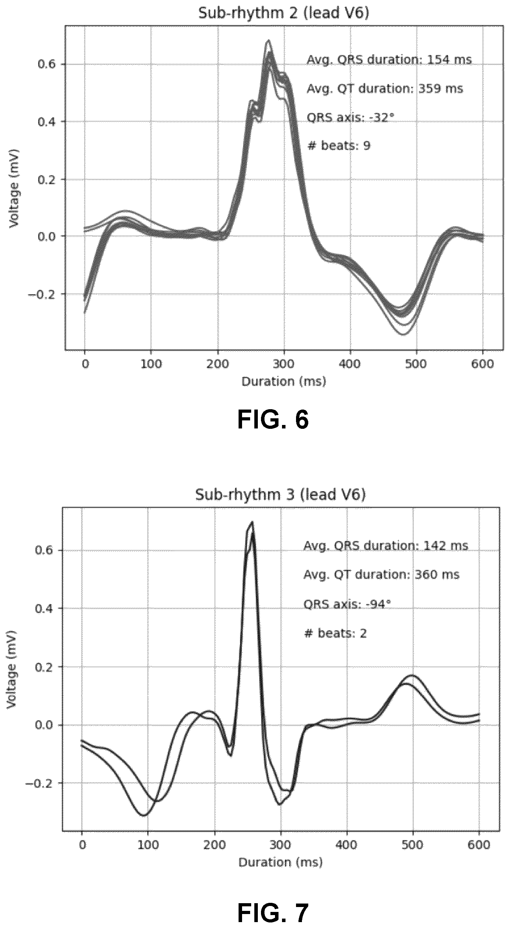

Fig. 5 shows all beats of the sub-rhythm 1 of the ECG signal of Fig. 3, together with their aggregated measurements. These beats present an Incomplete LBBB pattern as we can see thanks to the average QRS duration and morphology. Their QRS axis is very similar to the beats shown in Fig. 6, which supports their similar origin, i.e. supraventricular with a block of varying degree in the left bundle branch.

Fig. 6 shows all beats of the sub-rhythm 2 corresponding to the ECG signal of Fig. 3, together with their aggregated measurements. These beats present a Complete LBBB pattern as we can see thanks to the average QRS duration and morphology. Their QRS axis is very similar to the beats shown in Fig. 5, which supports their similar origin, i.e. supraventricular with a block of varying degree in the left bundle branch.

Fig. 7 shows the 2 isolated PVCs corresponding to the ECG signal of Fig. 3, with their aggregated measurements. These beats present a QRS duration like the Complete LBBB ones (cf. Fig. 5), but their QRS axis is very different and extremely deviated. This supports the diagnosis that these 2 beats are coming from a very different location in the heart, the ventricles.

The table below shows a comparison between the aggregated measurements corresponding to the conventional method and the measurements stratified by sub-rhythms in accordance with the invention.

| | QRS duration | QT duration | QRS axis | Nb of beats |
|---|---|---|---|---|
| All beats (standard of care) | 134 ms | 360 ms | -41° | 21 |
| Sub-rhythm 1 | 110 ms | 322 ms | -41° | 10 |
| Sub-rhythm 2 | 154 ms | 359 ms | -32° | 9 |
| Sub-rhythm 3 | 142 ms | 360 ms | -94° | 2 |

It can be seen that the conventional aggregation method hides significant differences between sub-rhythms even within a short 10-second ECG. For example, the conventional method hides the fact that there are 2 dominant rhythms in this ECG: sinus rhythm with Incomplete Left Bundle Branch Block LBBB, and sinus rhythm with complete LBBB. There are finally two isolated premature ventricular complexes PVCs, which could easily be taken for beats with a Complete LBBB pattern by just looking globally at the ECG.

The method in accordance with the invention allows for a more accurate and fine-grained understanding of the measurements of a given ECG, which in turn guides the user towards a better diagnosis of the ECG. The decomposition of the ECG signal into sub-rhythms gives a better representation of the ECG to the user. In the above example, displaying measurements for each identified sub-rhythm helps the user to understand more easily a complex ECG signal, by highlighting similarities (QRS axis for the sub-rhythms 1 and 2) and differences (QRS axis between sub-rhythms 1 and 2 versus 3, and QRS duration between sub-rhythm 1 and 2) between sub-rhythms.

The above-described method and system may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for facilitating the diagnostic of cardiac abnormalities based on electrocardiogram ECG measurements, the method comprising the steps of:
- receiving an ECG signal;
- analyzing the ECG signal using a delineation algorithm so as to segment the ECG signals into multiple beats;
- determining for each beat a set of measurements;
- grouping beats having similar measurements per cluster, each cluster representing a different sub-rhythm;
- aggregating measurements within each cluster before being displayed to a user.

2. The method of claim 1, wherein the method further comprises the step of applying an embedding algorithm to generate embedding data representative of the plurality of beats, and where the grouping step is based on beats having a similar morphology.

3. The method of claim 1 or 2, wherein measurements include one or more of:
- QRS duration;
- QRS axis;
- QT duration;
- corrected QT duration;
- PR duration ;
- ST elevation;
- RR with preceding beats ;
- P wave axis ;
- P wave duration.

4. A computer program comprising code means for implementing the method of any preceding claim when said program is run on a processing system.

5. A system for facilitating the diagnostic of cardiac abnormalities based on ECG measurements, the system comprising an input interface configured to:
- receive an electrocardiogram ECG signal;
- analyze the ECG signal using a delineation algorithm so as to segment the ECG signal into multiple beats;
- determine for each beat a set of measurements;
- group beats having similar measurements per cluster, each cluster representing a different sub-rhythm; and
- aggregate measurements within each cluster before being displayed to an user.

6. The system of claim 5, wherein the user interface is further configured to apply an embedding algorithm to generate embedding data representative of the plurality of beats, and where the grouping step is based on beats having a similar morphology.

7. The system of claim 5 or 6, wherein measurements include one or more of:
- QRS duration;
- QRS axis;
- QT duration;
- corrected QT duration;
- PR duration ;
- ST elevation;
- RR with preceding beats ;
- P wave axis ;
- P wave duration.

8. A monitoring system comprising a cardiovascular monitor and the system of claim 5.
